# EUROPEAN PATENT APPLICATION

(11) **EP 2 486 955 A1**
(43) Date of publication of application: **15.08.2012**
(21) Application number: 11158423.1
(22) Date of filing: 16.03.2011
(51) Int. Cl.: A61N 5/06

(54) **Therapeutic cap**

(30) Priority: 10.02.2011 BR MU9100002 U
(71) Applicant: Pereira De Oliveira, Alvaro, 01409-000 Bela Vista SP (BR)
(72) Inventor: Pereira De Oliveira, Alvaro, 01409-000 Bela Vista SP (BR)
(74) Representative: Zanoli, Enrico

(57) **Abstract**

A therapeutic cap (1) is disclosed, comprising a body (2) in the form of a cup or spherical cap, from whose front end a visor (3) projects; wherein the end of the body (2) has an edge (4); the back portion of the edge (4) may include an elastic element, an adjustable strap or any conventional device to adapt the circumference of the cap to the diameter of the user's head. Said cap (1) is made of a multilayered material, said material comprising at least one external finishing layer (9), one structural intermediate layer (10) and one internal reflection layer (11). To the internal part of the body (2) of the cap (1), strips (5) of red LEDs (6) are affixed to irradiate the scalp of the user. Due to the healing, microcirculation stimulating and analgesic properties of the red light emitted by the LEDs, said cap may also be used as a complement in other scalp pathologies, such as alopecia in general (androgenetic, areata, traction, etc.), folliculitis, psoriasis and other inflammatory pathologies in the region.

## Description

The present invention refers to a therapeutic cap, more specifically, to a cap provided with internal LEDs intended for hair treatment.

### BACKGROUND OF THE INVENTION

Low intensity red light has been used to help in the treatment of various health problems. As an example, low intensity red laser is used for better wound healing, low power red laser to reduce pain in dentistry, low power red laser to improve the skin appearance, comb shaped red laser to stimulate hair growth, yellow laser and LEDs to improve skin appearance, blue lasers and LEDs for acne treatment. Specifically concerning hair treatments intended to improve hair quality, it is known that red light (620-630 nm) has healing, microcirculation stimulating and analgesic properties, among others. Such properties have been widely used, e.g. for the treatment of androgenetic alopecia (hair loss usually following men's aging), and as a complement in the treatment of other scalp pathologies, such as alopecia in general (areata, traction, etc.), folliculitis, psoriasis and other inflammatory pathologies in the region. Practically speaking, a few devices intended to implement such characteristics in the scalp are known in the art. Among these documents, we can mention GB 222,963, published on October 13, 1924, disclosing a piece of equipment for hair treatment comprising a box with an opening for the entrance of the user's head. The box of the equipment is provided with a high tension electrical discharge ozone generator and color lamps for hair treatment (blue and red) inside it. The device comprises a rectangular box with an open lower front part. The hair is placed on a sloped wall. An induction coil in housing is connected to electrodes projecting inside the box. Electrically heated wires are provided, as well as red and blue lamps, each one turned on by an independent switch, a cooler, a door and a thermometer. Despite the number of treatments that may be carried out by the equipment, its size is large, which makes it incompatible with domestic use.

Within the scope of an object with lower dimensions, and therefore easy to buy and to use by the user, the state of the art provides, just like disclosed by document CN1398650, published on February 26, 2003, a hairbrush provided with red LEDs to improve blood circulation and hair growth, and to reduce itching. In a similar way, PI 0704946-3 discloses a LED brush for hair treatment by phototherapy. The object is to supply a brush with light emitting diodes for hair treatment by phototherapy. Such treatments are against baldness and color loss. The also increase hydration in order to improve life and health of individuals. The device is composed of a module which is 10 cm wide, 20 cm long and 5 cm high. At the end of the application module, there is a small brush provided with ten LEDs, red with wavelengths of 650 nm and blue with wavelengths of 405 nm. The device also includes an operation switch and is powered by internal lithium batteries. The user should turn on the device and brush or comb the hair, covering the whole scalp. The length of the treatment session is defined by the patient himself/herself, but daily use for at least 15 minutes is recommended.

Another similar solution is disclosed in PI 0802844-3, comprising an electric plate or LED and ultrasound brush for hair treatment, whose purpose is to supply an LED and ultrasound brush for hair treatment by phototherapy and ultrasound. Such treatment is hydration and smoothening, against color loss and, it aims at improving life and well-being of individuals. The device is composed of a module which is 20 cm wide and 10 cm long and a flat cable which is 10 cm long and 5 cm wide. In the application module, there are two small plates, provided on one side with 20 LEDs, in red and blue colors, with high shine and wavelengths of 630 nm and 405 nm, respectively. On the other end, an ultrasound plate is provided to stimulate water molecules, and thus, the hair.

Despite the fact that the three solutions above are effectively compact and easy to use, all of them present a common problem, which is the lack of control of the time of exposure of the scalp to the treatment proposed. In these cases, a more precise control of exposure time of a given treatment area is very difficult, since these devices are placed on the user's head. Thus, one area may receive much more irradiation than another, causing inconsistent results. Furthermore, it is doubtful whether a user will spend continuous fifteen minutes combing his/her hair, day after day, no matter how light the device is.

Another solution that eliminates the need of "handling" by the user refers to the use of hats and caps, which are placed on the user's head and taken off at the end of the treatment. Document JP 8092810, published on April 9, 1996, discloses a cap designed for children to produce a comfort effect by an internal red light reflecting chamber made of textile material. There is no reference to any capillary properties.

Finally, the art also discloses three other very similar solutions for the use of caps in capillary treatments. Among them, document JP 2002 129424, published on May 9, 2002, refers to a hat intended to revitalize weak hair roots due to the constant use of hats and caps; the hat is red and allows IV rays to pass through it, being also indicated for the treatment of alopetia areata. On the other hand, document JP 2006 037318, published on February 9, 2006, refers to a hat that only allows the passage of red spectrum light rays; in this case, the rays reach the scalp and activate the hair roots, stimulating capillary growth. Finally, document WO 2008/069154, published on June 26, 2008, discloses a hat that allows red light rays near IV to penetrate, to promote the hair growth and reduce dysfunctions of brain vascular origin. Despite overcoming the previous proposals and effectively solving user's handling problems, none of these solutions is free from inconveniences, since, in these cases, the cap is simply put on and nothing more. Said solutions act as light filters and particularly as sunlight ray filters. Thus, the use of said caps inside houses is highly compromised, be it for insufficient lighting in the environment, or for the illumination generated by narrow spectrum light sources (e. g. cold lamps).

### SUMMARY OF THE INVENTION

Therefore, a first scope of the present invention is a device intended for capillary treatment, by means of light irradiation within the red spectrum, which is portable, easy to use and inexpensive.

Another object of the present invention is to provide a device intended for capillary treatment by light irradiation within the red spectrum, which may be used anywhere indistinctively, as well as independently from external lighting sources.

A further object of the present invention is to provide a special cap comprising internally applied red LEDs, intended to stimulate blood circulation in the scalp and increase mitochondrial activity, stimulating hair proliferation and making them stronger and with better appearance.

These and other objectives are reached by the device proposed herein, which comprises a body in the form of a cup or spherical cap, from whose front end a visor projects, wherein the end of the body also has an edge, and the back portion of the edge may include an elastic element, an adjustable strap or any conventional device to adapt the circumference of the cap to the diameter of the user's head. Said cap may be made of a multilayered material, with said material comprising at least one external finishing layer, one intermediate structural layer and one internal reflection layer; wherein in the internal part of the cap body, at least one LED strip is placed and fastened to it, being each LED in the strip powered by a cable connecting the strip to an energy source.

### DESCRIPTION OF THE FIGURES

The object of the present invention will be better understood in the light of the following detailed description of one of its preferable embodiments, which is an example and is made based on the attached drawings, wherein:

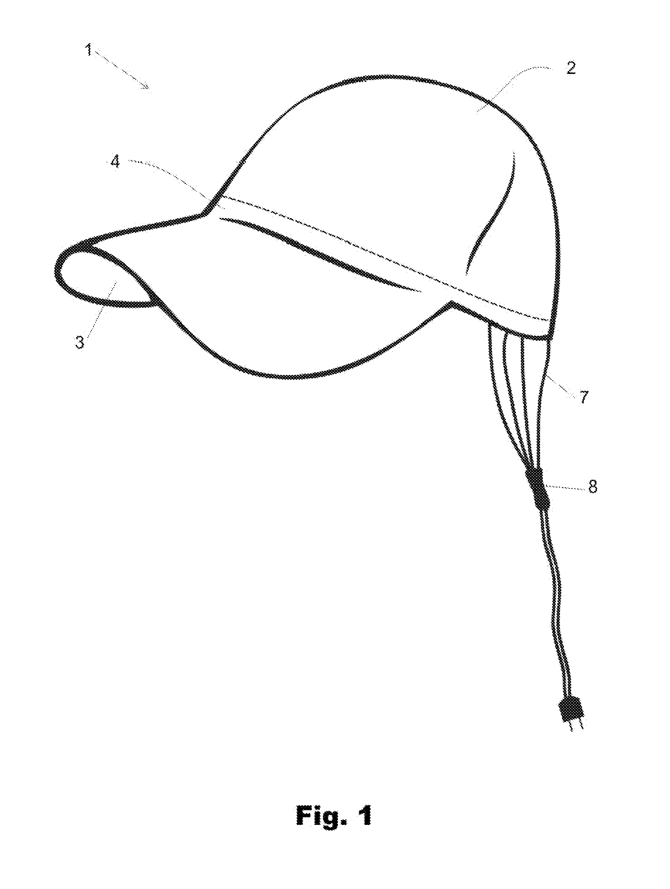

- Figure 1 is a front perspective view of the cap of the present invention;
- Figure 2 is a lower view of the object of Figure 1, showing the internal arrangement of LEDs; and
- Figure 3 is a cross section view of the object of Figure 1, showing the component layers of the cap.

### DETAILED DESCRIPTION

According to the attached drawings, the object of the present invention comprises a cap, jointly indicated by 1, composed of a cup shaped body, from whose front end a visor 3 projects. The end of the body 2 also presents an edge 4, generally formed by folding the material composing the cap 1, said fold involves a laminar structure (not seen) usually made of plastic or similar material, to provide a structural function to the cap and to fit the cap 1 to the user's head. Alternatively, the back portion of the edge 4 may have an elastic element, an adjustable strap or any conventional device to adapt the cap circumference to the diameter of the user's head.

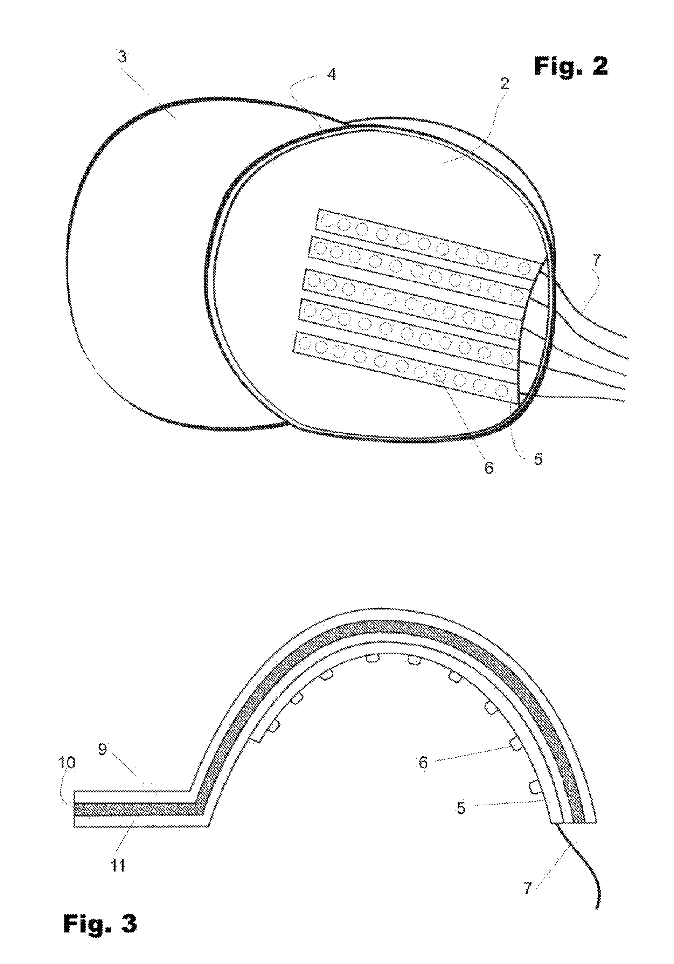

The device itself, and in accordance with to the invention at issue, basically comprises a series of LED strips 5 placed lengthwise inside the body 2 of the cap 1 (specifically refer to Figure 2). Each one of said LED strips 5 has a given number of aligned LEDs 6, electrically connected in parallel. Each one of said strips 5 is powered by a cable 7 with reduced diameter, which is able to deliver appropriate tension to supply all LEDs 6 placed on the corresponding strip 5.

Preferably, each one of the LEDs 6 is a red LED (wavelength 620-630 nm) with less than 0.1 W power, but said feature is not preponderant, since the full amount of energy supplied (ratio: number of LEDs x individual power) should be maintained. Therefore, the total energy will be kept with the increase in power of each LED, as long as the number of LEDs used is reduced, and vice-versa.

Furthermore, the arrangement of the strips 5 may widely vary in relation to the illustration in Figure 2, depending on the region of the scalp which should be illuminated, and therefore depending on the pathology/condition to be treated. An alternative arrangement for the strips 5 comprises one or more lengthwise strips and a circumference strip in closed ring (or almost closed) and approximately parallel to the edge 4 of the cap 1, which results in a similar structure to bicycle rider's helmets (radiated spherical cap).

In a first embodiment of the present invention, each one of said cables 7 is connected to a transformer 8 (9~12 V_{DC}), which is then connected to a home outlet (not shown) (110∼220 V_{AC}). In an alternative embodiment, each cable 7 is connected to a conventional 9V battery (not shown), by means of an appropriate plug (not shown). In this alternative embodiment, the battery may be fastened to the edge 4 in a conventional way (a pocket formed on the edge, internally, to an elastic strap transversely fixed to the edge, etc.).

An important characteristic of the cap 1 is the shape of the body 2 (in the form of a cup or spherical cap), so that, when the user puts on the cap 1, the whole top of the body 2 (region where the strips 5 and, therefore, the LEDs 6 are located), is not in contact with the user's head, maintaining a distance from the user's head and the strips 5. Said distance between the user's head and the LEDs 6 allows the light emitted by the LEDs 6 to be better irradiated, in terms of the coverage area to be treated.

In order to obtain said shape and to hold the body 2 of the cap so that it does not collapse over the user's head during use (due to the weight of the strips 5), the material forming the cap 1 is a multilayered laminar material. More particularly, and as schematically shown in Figure 3, the material of the cap 1 comprises at least one external layer 9, known as the finishing layer, a core layer 10, known as the structural layer, and an internal layer 11, known as reflection layer.

The external layer 9 may be for example a polyester textile layer, such as usually used in conventional caps, or made of a similar material. The core layer 10, or structural bed, is formed by interfacing fabric, which is a usual material employed in the clothing industry to improve the stiffness of certain parts of clothes (e. g. collars of dress shirts for men). The interfacing fabric, as known, is affixed to the textile by the application of heat (100 - 130° C) for a set time period (10-20 seconds). As a result, both the material of the external layer 9 and the material of the internal layer 11 should support such a temperature range with no degradation or deterioration.

Finally, the internal layer 11 is a layer made of a reflexive material, intended to reflect the light emitted by each LED 6 on the head of the user. We stress that the cup spherical shape of the body 2 naturally helps to concentrate the light on the user's head, as the reflector panel of a headlight, or optical group, of a vehicle. The layer 11 is made of a light reflecting textile and in particular a shining silver textile which is highly reflexive such as the ones used in photography, so that maximum light is reflected to the scalp.

In use, the user puts on the cap 1 and turns it on, so that the LEDs 6 light up and emit a given amount of light on his/her scalp. Due to the internal layer 11, most light emitted by the LEDs 6 is directed to the user's head, which minimizes the loss of power supplied.

An already tested example of the cap, according to the present invention, includes 60 LEDs located in such a way that the five regions most affected by androgenetic alopecia (hair loss usually following the men's aging) receive an appropriate quantity of light. Said regions are called vertices, front region, central region and sides. Said 60 red LEDs, with a full power of about 4.3 Watts, are distributed in a semicircular shape at the front and side regions. Said configuration guarantees a transfer of at least 60 J/cm² of irradiated scalp area after about 15 minutes of use. Said intensity in energy transfer is within the currently adopted limits for LLLT (Low Level Laser Therapy) treatments, which are between 10 and 60 J per session. In a particular case, just 10 minutes of daily use of the cap (10 minutes / 9 V / P_{LED} = 0.05 W => 18 J per LED, i. e. ≈20 J/cm² irradiated) produced very satisfactory results, within the standards of LLLT treatments.

Due to the healing, microcirculation stimulating and analgesic properties of the red light emitted by the LEDs, said cap may also be used as a complementary treatment in other scalp pathologies, such as alopecia in general (areata, traction, etc.), folliculitis, psoriasis and other inflammatory pathologies in the head.

## Claims

1. Therapeutic cap comprising a body (2) in the form of a cup or spherical cap, from whose frontal end a visor (3) projects; wherein the end of the body (2) presents an edge (4); wherein the back portion of the edge (4) may include an elastic element, an adjustable strap or any conventional device to adapt the cap circumference to the diameter of the user's head, **characterized by** said cap (1) being made of a multilayered material, said material comprising at least an external finishing layer (9), a intermediate structural layer (10) and an internal reflection layer (11) and, having in the internal part of the body (2) of the cap (1), at least one strip (5) of LEDs (6) fixed to the body (2), being each LED (6) of the strip (5) powered by a cable (7) connecting the strip (5) to a source of energy.

2. Therapeutic cap of claim 1, **characterized by** said at least one cable (7) being connected to a tension transformer (110∼220 V_{AC} 9-12 V_{DC}).

3. Therapeutic cap of claim 1, **characterized by** said at least one cable (7) being connected to a battery (9 V_{DC}), being said battery fastened to the edge (4) of the cap (1).
